(19) Europäisches Patentamt

European Patent Office

Office européen des brevets

(11) **EP 1 688 746 A2**

(12) **EUROPEAN PATENT APPLICATION**

(43) Date of publication:
09.08.2006 Bulletin 2006/32

(51) Int Cl.:
*G01P 7/00* (2006.01)   *G01P 1/12* (2006.01)
*A63B 24/00* (2006.01)

(21) Application number: 06425050.9

(22) Date of filing: 01.02.2006

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 08.02.2005 IT GE20050008

(71) Applicants:
• S.I.T. S.r.l.
56127 Pisa (IT)
• Verza, Andrea
56127 Pisa (IT)

(72) Inventors:
• Facchielli, Duccio
50139 Firenze (IT)
• Torti, Stefano
50139 Firenze (IT)
• Verza, Andrea
50053 Empoli (FI) (IT)
• Chiarugi, Antonio
56127 Pisa (IT)
• Clot, Paolo Rosa
56127 Pisa (IT)
• Clot, Marco Rosa
56127 Pisa (IT)

(54) **Apparatus for monitoring human body's movements in sport or medical domain, and its use**

(57)     A precise measurement of human body's movements is highly important in all those domain where monitoring such movements is essential to: screening the effort made during a movement, analyzing human limits in performing such movements, train and improve human abilities of movement performance. Such context are easily found in the rehabilitation domain and/or physical training. This immediately leads to physiotherapy and sport. However, current methods (especially in sports) are extremely expensive because they involve the use of cameras and ultrasound acquisition systems. A widely used system uses accelerometers to assess acceleration, and mathematically deduct velocity, strength, and power of a movement. But, a serious limitation of all the already patented inventions using accelerometers and of their mechanism is that in no case they obtain a precise estimation of the monitored object (or a part of it) motion. The current industrial invention is, indeed, a new invention relating to a new system for estimating any body part displacement -and, therefore, motion—without any external reference, by means of inertial measurement. Furthermore, the proposed new method, unlike the potential competing technologies allows a very precise estimation of monitored motions.

Picture 4

**Description**

**Technical division (WIPO classification):**

G01P 7/00: Speed measurement through acceleration integration and distance measurement through acceleration double integration.

**State of the Art:**

A precise measurement of human body's movements is highly important in all those domain where monitoring such movements is essential to:

- screening the effort made during a movement
- analyzing human limits in performing such movements
- train and improve human abilities of movement performance

Such context are easily found in the rehabilitation domain and/or physical training. This immediately leads to physiotherapy and sport. However, current methods (especially in sports) are extremely expensive because they involve the use of cameras and ultrasound acquisition systems. In addition, the latter are bulky and they are extremely limited for they force the athlete to perform inside a given reference system. This is a particularly serious limitation for all outdoor sports where, by definition, the athlete cannot perform inside a lab. Trying to solve these limitations, industry has recently tried to develop less expensive human body monitoring systems by eliminating every expensive and sophisticated recording device or data acquisition, and sing instead simple devices, independent from the reference systems.

A widely used system uses accelerometers to assess acceleration, and mathematically deduct velocity, strength, and power of a movement. Several companies developed simple instruments (consider, for example, products by *Intersense Inc.* or *Timex*) to be wrist worn, like a watch, to allow performance monitoring during sports. Product such as those are subject to an intense research activity both in private companies, and universities, especially Americans'. *Intersense* products, are the result of Eric M. Floxin's development aand research activity at the *Massachusetts Institute of Technology* , Cambridge, MA (USA) at first, and then at *Intersense* labs. He is the author of the two patents on head's inertial movement tracing by the way of calculation estimating the movement relative to a moving platform (1, and 2), and of the very recent patent (end of June 2004) on tracing "without reference", using just head orientation (3) in virtual reality applications. In September 2004 he also got license for another patent (4) introducing in the system a second sensor measuring inclination variations as to Earth's gravitation , or variations in rapport to Earth's magnetic field to obtain a rotation correct estimation. In this patent, to avoid the problem of deviation compensation in orientation estimation, Floxin include the possibility of using a Kalman filter based on human movements obtained on the base of statistical data. Such a problem is similar to he one solved by the industrial solution—related to deviation in displacement estimation—that Floxin partially explored at MIT (5) taking into account periodic moments of head's rest and motion (6). As a matter of fact, Floxin already dealt with this theme in the past: in 1997 he developed a method for integrating signals, acquired via a differential sensor, in time (7); he also developed methods that took into account motion and resting characteristics (8). All those works were directed to human head's movement traceability. Others have used gravitational or magnetic field measurements to estimate special orientation (see, for example, reference (9)), but to Floxin undoubtedly goes the merit of applying such methodologies to human head's orientation estimation in virtual reality applications. There are more than 200 worldwide patents on *motion tracking* related topics., such as the one by the Korean company LG Electronics Inc. (10), or the one by SAMSUNG Electronics Co Ltd (11). All those patents are based on motion reconstruction starting video recordings and/ or image segmentation methods (12). On the contrary, there are only four patents on traceability of human body motion: two American, one German, and one Korean. The first American patent is by XEROX Corp. on human head marking in video (13); the German one is the request in Germany of a U.S. patent; there is no news about the Korean patent (14)—and considered the date of the patent (the request date is 2001) we can assume it had poor practical and industrial application. The second American patent is more relevant to the invention presented in this patent: it is a 1997 *Stanford University* patent, presenting a method for estimating motion integrating (15). However, this patent is relating to the reconstruction of a human hearth motions, starting from images acquired through Magnetic Resonance. The proposed computing method is based on the assumption of always being in presence of periodic motions, and, therefore, be able to estimate motion from Fourier harmonics, obtaining trajectory estimations not subject to deriva, even in of high noise conditions. This very patent, quotes other methods for estimating trajectories for periodic motions, such as the "direct and inverted integration" (16), or the "force closure" one(17). Those methods assess the errors cumulating with the velocity estimations and correct them, improving trajectory estimations. Another quoted method is the iterative one, recursively calculating portions of trajectory aiming at refining it in order to improve the trajectory evaluation, or alternatively an iteration approach, evaluating step by step the trajectory with the target of approaching better the estimated velocity to the effective local velocity using as starting point the phase contrast data in a velocity field (18). A serious limitation of all these inventions and of their mechanism is that in no case they obtain a precise estimation of the monitored object (or a part of it) motion. In particular, all the aforementioned methods tend to overlook motion because of the limits of their integration models. Furthermore, an assessment of these patents' application range shows how they are all related to applications on the field of trajectory estimation from Magnetic Resonance (18), and, in particular, in the case of the study of cardiac movements (17), or other internal organs (16). Finally, the last document to be considered, relating to displacement estimation is connected to a recent request of American patent (still pending) by Daniel M. Iaria and David M. Torsi. This patent is based on a measurement (obtained with a heat sensor), of the heat trace left by an object with a heat source attached, and moving on a surface (19). The current industrial invention is, therefore a new invention relating to a new system for estimating any body part displacement--and, therefore, motion—without any external reference, by means of inertial measurement. Furthermore, the proposed new method, unlike the potential competing technologies allows a very precise estimation of monitored motions.

**Invention description:**

In the apparatus related to the presented industrial invention, the acceleration measurement is made an accelerometer. The new method here suggested, can of course be applied to all the three special dimensions, but to simplify the description, we will illustrate only the one-dimensional case. In this case we can think of having a monoaxial accelerator, located on the body part to be monitored. Both in case of sport, and medical application the accelerometer is mounted in a device that can easily be worn (such as with a strap), to measure the acceleration of a body part (arm, hand, foot, head, elbow, or any other body part allowing independent motion). In any case, monitored motions will be of a repetitive kind, like in case of a weight training session, walking, jumping, running, or physiotherapy. Motion will, therefore, start from resting position to return periodically back to resting position with zero speed. The accelerometer will measure the instantaneous acceleration, and motion is reconstructed by means of the usual cinematic equations:

$$v(t) = v_0 + \int_0^t a(t)dt \qquad (1)$$

$$z(t) = z_0 + \int_0^t v(t)dt \qquad (2)$$

Initial speed is zero and the initial position is known. Of course, the initial position depends on the initial position of the body, and the latter can be measured by other means, with external additional information on the motion, or can also remain unknown since the most important information is, usually, the motion related to the starting position.

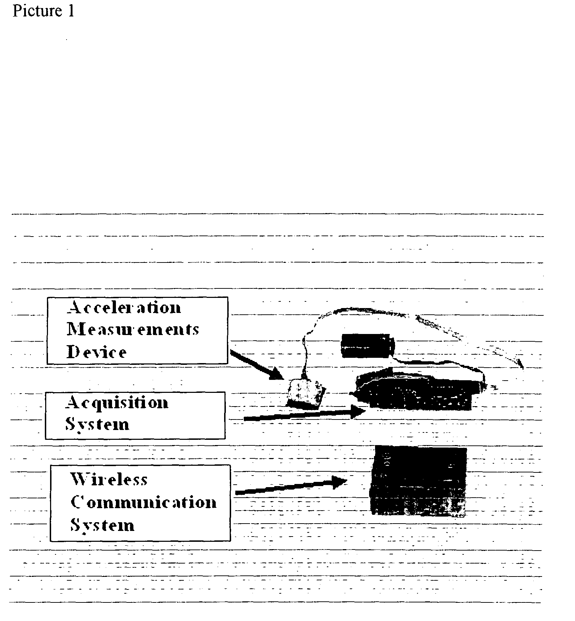

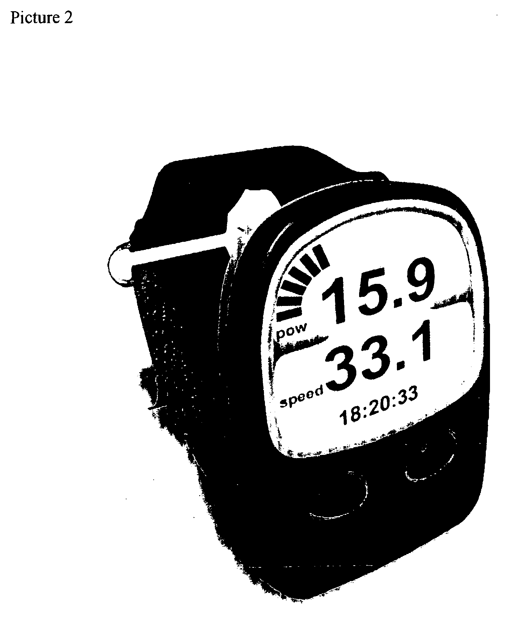

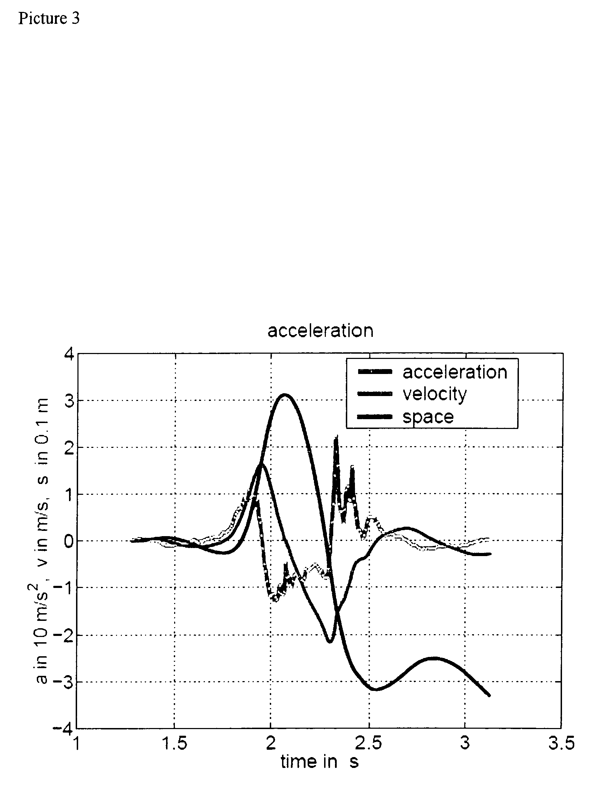

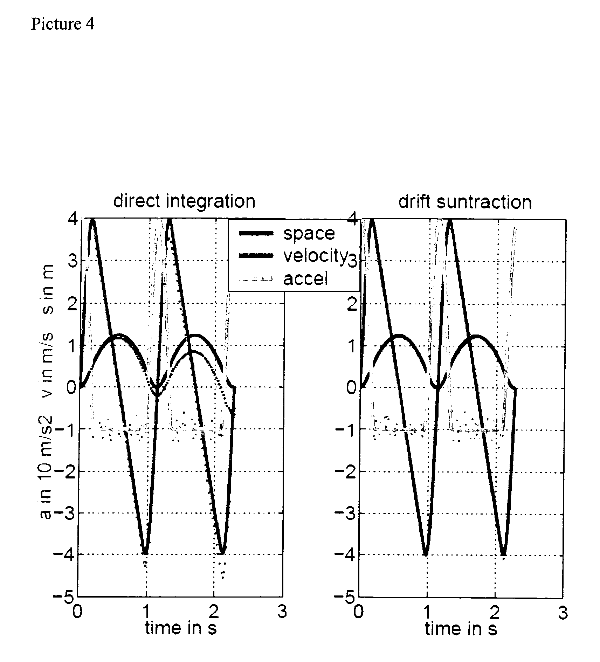

It must be noted that, since the system can be applied to body motions, monitored motions are within half a meter and with executing timers in the range of few seconds. In the current invention I therefore possible to think both to a system acquiring the data, store it in a computer performing the off-line calculation, and send back the data on the user's demand (fig. 1), or to an on-line system providing the information at the end of every set of motions (fig. 2).

**The Problem.**

In case of an acquisition and real elaboration based on the aforementioned equations (1) and (2), the usual result is similar to the one described in picture 3 (a jump). The picture reports the acceleration measured with the accelerometer, the speed measured with equation (1), and the displacement measured with equation (2). Note how marked is the estimation error on the displacement (the final position is inferior to the initial one, while it should be the same). The reason for these errors is rather obvious: it is due to the accumulation in the mathematical integration process of the acceleration measurement statistical errors. Picture 3 clearly shows how such an accumulation generates estimation errors, both of position (inferior to what it should be, knowing , that it describes a jump on the spot), and of velocity (this should be zero, at the end of the jump, while the system register some velocity). This is exactly the same problem Eric Floxin and his colleagues working on trajectories in Magnetic Resonance biomedical images confronted for years: correct estimation deviation.

**The New Method.**

To solve the problem of correct estimation deviation, the present industrial invention suggests a new method, introducing a new way of dealing with correct estimation from stochastic drift. Stochastic drift average value can be easily calculated (20) and connected both to experimental measurement error, and to sampling frequency through the equation

$$\sqrt{\Delta x^2} \sim \frac{\sigma_a}{\nu} \tag{3}$$

In principle, this equation can be read as a suggestion to reduce derivation by a reduction of the measurement error, or by augmenting the sampling frequency of the signal. However, such a solution is not always practicable because the measurement error is already the smallest one given by the employed accelerometers, and the sampling frequency cannot be freely augmented.

In the present industrial invention, this problem is solved introducing in the calculation method supplementary information, obtained from the knowing the typology of the screened motions. In the case of repetitive motions, we can assume that:

Initial and final speed of each repetition is zero.
Initial and final position are the same in each repetition (within eventual measurement errors).
These two information on motion can be translated into two restrains for the calculation system of motion and speed estimation introducing the following equations:

$$\int_{t_{in}}^{t_{fin}} a(t)dt = 0 \tag{4}$$

$$\int_{t_{in}}^{t_{fin}} v(t)dt = 0 \tag{5}$$

Since measured acceleration does not satisfy these two prerequisites because of the stochastic drift, it is possible to force it by subtracting from the acceleration a function $\Phi(t)$ satisfying the following equations:

$$\int_{t_{in}}^{t_{fin}} \phi(t)dt = \int_{t_{in}}^{t_{fin}} a_{exp}(t)dt \tag{6}$$

$$\int_{t_{in}}^{t_{fin}} \int_{t_{in}}^{t_{fin}} \phi(t)dt = \int_{t_{in}}^{t_{fin}} \int_{t_{in}}^{t_{fin}} a_{exp}(t)dt \tag{7}$$

Such a function can be written in various forms, but the simplest is a linear one $\Phi(t) = \alpha + \beta t$, where constants $\alpha$ e $\beta$ satisfy two equations derived from the former (6) and (7):

$$\alpha \cdot (t_{fin} - t_{in}) + \frac{1}{2}\beta \cdot (t_{fin} - t_{in})^2 = \int_{t_{in}}^{t_{fin}} a_{exp}(t)dt \qquad (8)$$

$$\frac{1}{2}\alpha \cdot (t_{fin} - t_{in})^2 + \frac{1}{3}\beta \cdot (t_{fin} - t_{in})^3 = \int_{t_{in}}^{t_{fin}} \int_{t_{in}}^{t_{fin}} a_{exp}(t)dt \qquad (9)$$

At this point, the function to be integrated is not integrated speed, but quantity

$$a_{exp} - \phi(t) \qquad (10)$$

In other words, the acquired acceleration data must be modified according to equation (10) before being able to use equation (1) and (2) to estimate displacement speed. As a manner to double check the good functioning of the presented method in this industrial, picture 4 reports the result of speed and displacement estimations from an acceleration simulation on the behavior of an ideal spring in condition of noise noisy background?. On the right side of the picture are the displacement estimations obtained comparing the present method results with the ones obtained with direct integration of equations (1) and (2) with acceleration as measured with an accelerometer..

## Expected Advantages of the Industrial Invention:

The present industrial invention proposes, for the first time, a gauge for displacement measure to be applied in all cases of repetitive motions (in training, competitions, or physiotherapy) monitoring. Such monitoring is extremely important whenever not only power measurement, or other magnitudes that can easily be obtained from an inertial measurement of acceleration, but motion accuracy is essential in measuring the athlete's (or the patient's) progression. Moreover, the system proposed in this patent has further advantages because:

the system is simply based on an accelerometer and does not need sophisticated apparatus, or external references;
the system component to b fixed on the athlete or on the patient is very small and very light, and, therefore, does not impair movements, or alters physical effort;
the proposed system can estimate displacement in repetitive exercises within 2% error of special accuracy.

## Bibliographical Reference:

American patent n. US **6,474,159**
American patent n. US **6,681,629**
American patent n. US **6,757,068**
American patent n. US **6,786,877**
American patent n. US **6,361,507**
American patent n. US **6,162,191**
American patent n. US **5,807,284**
American patent n. US **5,645,077**
American patent n. US **6,820,025**
Korean patent n. **KR2003073879**
Korean patent n. **KR2003060328**
Spanish patent n. **EP1411474**
American patent n. US **5,802,220**
Korean patent n. **KR2002084910**
American patent n. US **5,615,677**

American patent n. US **5,257,626**
American patent n. US **5,257,625**
American patent n. US **5,680,862**
American patent request n. **20020118170**
J. M. Thong, M. S. Woolfson, J. A. Crove, B. R. Hayes-Gill and
R. E. Challis Meas.Sci.Technol. 13 (2002) 1163-1172.

## Claims

1. An apparatus for displacement measure, including: a system for acceleration inertial measures; a system for acceleration measurements storage; a computing system for the estimation of velocity, displacements, and other derivate quantities; a system for the storage of speed and displacement estimations storage.

2. An apparatus for displacement measurement, as per claim number 1, where the system is made with a low energy consumption system.

3. An apparatus for displacement measurement, as per claim number 1, where the system for the measurement of acceleration is formed by one or more accelerometers with one or more axis.

4. An apparatus for displacement measurement, as per claim number 1, where the system for the measurement of acceleration storage, the computing system for the estimation of velocities and displacements, and the system for the storage of velocities and displacements estimation has a different housing of the system for acceleration inertial measurement.

5. An apparatus for displacement measurement, as per claim number 1, where the system for the measurement of acceleration storage, the computing system for the estimation of velocities and displacements, and the system for the storage of velocities and displacements estimation has the same housing of the system for acceleration inertial measurement.

6. An apparatus for displacement measurement, as per claim number 5, where the computing system performs "off-line" computations, that is the computing system performs all the calculations needed for the estimation of speed, displacement, and derivate quantities after all the movements have been performed, and all their accelerations have been recorded in the acceleration storage system.

7. An apparatus for displacement measurement, as per claim number 7, where the system for the storage of velocities and displacement estimations records the estimations and feed them back only after user's request.

8. An apparatus for displacement measurement, as per claim number 6 where the computing system performs computations immediately after every motion repetition and immediately provide the result.

9. An apparatus for displacement measurement, as per claim number 9, where the system for the storage of velocities and displacement estimations records the estimations and feed them back after user's request.

10. An apparatus for displacement measurement, as per the claim number 1, where the computing system for velocity and displacement estimations includes a computing system via integrations of measured acceleration.

11. An apparatus for displacement measurement, as per claim number 11 where the computing system for velocity and displacement estimations includes a computing system via elaboration of measured acceleration.

12. An apparatus for displacement measurement, as per claims number 11 and 12, where the computation system for velocity and displacement estimations includes a computing system via integration of the function derived from an elaboration of the measured acceleration.

13. An apparatus for displacement measurement, as per claim number 13, where the computation system for velocity and displacement estimations includes a computing system via integration of a linear function, derived from an elaboration of the measured acceleration.

**14.** An apparatus for displacement measurement, as per claim number 14, where the system is applied to the monitoring of motions related to jumping, walking, running exercises, or other free overloaded exercises, even with muscle development machines.

**15.** An apparatus for displacement measurement, as per claim number 14, where the system is applied to monitoring of fixed motions in sports.

**16.** An apparatus for displacement measurement, as per claim number 14, where the system is applied to monitoring repetitive motions in a medical context.

Picture 1

Picture 2

Picture 3

acceleration

Picture 4